# EUROPEAN PATENT SPECIFICATION

(11) **EP 0 252 227 B1**
(45) Date of publication and mention of the grant of the patent: **13.04.1994**
(21) Application number: 87105574.5
(22) Date of filing: 15.04.1987
(51) Int. Cl.: A61K 31/66

(54) **Use of inositol triphosphate in the treatment of metal intoxication**
Verwendung von Inositol-Triphosphat zur Behandlung von Metallvergiftung
Utilisation de l'inositol-triphosphate pour le traitement de l'intoxication par des métaux

(30) Priority: 16.04.1986 SE 8601709
(43) Date of publication of application: 13.01.1988
(60) Divisional application: 92107778.0
(73) Proprietor: PERSTORP AB, 284 80 Perstorp (SE)
(72) Inventor: Sirén, Matti, SF-00170 Helsinki 17 (FI)
(74) Representative: Hansen, Bernd, Dr. Dipl.-Chem.

(56) References cited:
- EP-A- 0 179 439
- EP-A- 0 179 440
- EP-A- 0 179 441
- BE-A- 903 497
- ALLGEMEINE UND SPEZIELLE PHARMAKOLOGIE UND TOXIKOLOGIE, 2nd ed., Bibliographisches Institut, Mannheim (DE); pp. 590-594#
- NEUROCHEM. INT., vol. 10, no. 3, 1987, Pergamon Journals Ltd. (GB); G.Y. SUN et al., pp. 361-369#
- MED. SCI. RES., vol. 17, 16-30 September 1989; C.J. GREEN et al., pp. 749-750#

## Description

The present invention relates to the use of inositoltriphosphate (IP₃) for the preparation of a medicament effective against conditions which are attributable to or caused by the presence of lead or mercury.

Even as early as the year 1900, different researchers had reported the finding of the organic phosphate compound phytic acid, i.e. 1.2.3.4.5.6-hexakis (dihydrogenphosphate) myo-inositol (also sometimes called inositol-hexaphosphoric acid) in plants. The content of phytic acid in different plants varies considerably. The content in grain is usually approximately 0.5-2%, with certain exceptions. Polished rice has a level of only 0.1% while wild rice contains as much as 2.2% phytic acid. Beans contain about 0.4-2%, oil plants approximately 2-5% and pollen 0.3-2%. The content of phytic acid in the plant varies during the growth period. The content is also influenced by, among other things, the climate.

In the literature there are reports on the presence of inositol pentaphosphate (IP₅) and inositol tetraphosphate (IP₄) in a few plants. It is further known that phosphate derivatives lower than IP₆ are formed at germination of grain. For instance the final products at the germination are inositol and phosphate. The use of IP₆ has been described in several scientific publications. The majority of the authors of these articles have observed several negative effects on humans and animals when consuming IP₆ or substances containing IP₆. Feeding dogs with too high an amount of IP₆ gives rise for example to rachitis. In humans lack of zinc and as a consequence thereof slower growth of children has been observed. Anemia has been observed mainly in women. Because of the above mentioned negative effects on the mineral balance in humans and animals, attempts have so far been made to reduce the intake of IP₆ and its derivatives to a minimum.

From C.A. Vol. 33 (1939), Abstr. No. 7351, No. 3/4 the use of phosphates including inositol phosphates as an anti-rachitic diet has been reported. No reference is made to specific inositol phosphates and nothing has been said in regard to complexing of metals.

U.S. patent 4,473,563 discloses the extra corporal treatment of erythrocytes to incorporate therein inositol phosphates to improve the oxygen supply. Then erythrocytes are separated from drawn blood which has been pumped out of the body for that purpose. After complicated treatment of erythrocytes the latter are reintroduced into the blood. There is no disclosure of administering inositol phosphates directly to the body. Moreover, nothing has been said in regard to treatment and alleviation of conditions caused or aggravated by the presence of lead, mercury, nickel or chromium in the body by a specially selected inositol phosphate.

In U.S. patent 2,723,938 the use of inositol phosphates is disclosed for stabilizing dispersions of acqueous suspension of penicillin. This ensures that brief simple manual shaking will restore a state of complete and uniform dispersion of the penicillin after prolonged storage.

BE A 903497, filed by the present Inventor, discloses pharmaceutical compositions containing IP₃, as the active ingredient, in the treatment of cadmium or aluminium intoxications or to reduce the formation of Free radicals.

For several hundred years metals in different forms have been used for instance at industrial processes. Early it was understood that certain metals, especially arsenic, lead and mercury are poisonous for humans.

The research relating to the physiological influence of metals on humans for instance has gone on all the time. It has got an increased importance during the last few decades, since people to an increasing extent are exposed to poisonous metals due to industrial discharges and other changes of the environment.

Many metals are necessary for the body. However, in too high concentrations these metals can give harmful effects. This case is valid for instance for iron, copper, zinc and magnesium. Thus, there is an indistinct limit between harmful and harmless/essential metals.

Non-essential metals can cause biological damages by interfering in biochemical processes. The metals or their ions can bind to biologically important molecules, negatively cooperate with enzymes and nucleic acids and influence the properties of cell membranes so that the normal function is disturbed.

To this group of metals firstly lead, mercury, nickel and chromium belong; and secondly arsenic, thallium, plutonium, barium, tin, copper and cobalt. In solution the metals are mainly present in the form of ions.

For natural reasons an acute supply of metals at a high concentration causes a temporarily increased resorption with acute damages on biological processes.

A long exposure to metals results in an accumulation in the organism, among other things in the tissues and a rather slow secretion of the metals. Thus, the effects of acute respectively chronical metal poisoning become different. Synergistical damaging effects can occur when the organism is exposed to several metals.

Lead poisoning is a very serious problem due to the rather usual presence of lead in the environment. Acute effects of lead poisoning can result in inflammatory damages, damages on the intestine, cardiovascular damages and shock conditions. The metals can also give rise to disorders in hormon glands, such as hypophysis, adrenal gland and thyroid gland. This fact is mostly evident for mercury. Chronical effects of lead poisoning can cause cardiovascular damages and hypertension but also brain damages and neurological disorders.

Poisoning caused by mercury can acutely give rise to kidney damages, liver damages, lung damages and damages on the intestine. Chronical effects are for instance mental damages, nerve disorders and effects on the immunity defence, which cause for example autoimmune diseases.

Moreover, it is previously known that the metals influence many enzyme systems, especially those containing thiol groups. Furthermore, the metals influence the metabolism in the second messenger system. Lead have in vitro caused a decrease of the DNA-synthesis.

Lead inhibits the enzyme xantinoxidase, while lead as well as mercury inhibit guanine aminohydrolase.

Metal chelates of different kinds, for example BAL (2.3-dimercapto propanol) and EDTA (ethylenediamino tetraacetic acid) have been used to cure or alleviate conditions caused by metal poisonings.

The chelates used so far have, however, negative properties, which has resulted in an insignificant clinical use. Thus, BAL is not soluble in water. Furthermore, it gives rise to a bad smell at the use. Its non-specific chelating ability also results in negative effects on the mineral balance concerning essential metals.

EDTA is resorbed to a low extent which is a limitation, since oral administration cannot be used.

Since the secretion velocity of this substance is high, rather high doses must be added to get an effect. However, this results in a disorder of the calcium level in the organism.

According to the present invention it has quite unexpectedly been found possible to solve the above mentioned negative effects of the toxic metals lead and mercury, on humans and animals and thus also to prevent or alleviate the connected diseases. Thus, the use of inositol triphosphate (IP₃), preferably in salt form has been brought about, for the preparation of a medicament effective against conditions which are attributable to, or caused by the presence of lead or mercury.

As examples of the diseases to which the use according to the invention refers, metal intoxication, allergy, inflammatory conditions, damage to the connective tissue, liver damage, brain damage, immunodeficiency, conditions of shock, gastroenteritis, dermatitis and neurological disturbances caused by any one of these metals, can be mentioned.

In addition the following diseases, when related with Hg or Pb intoxications, can be mentioned; cell proliferative changes, cancer, cardiovascular diseases, high blood pressure, damage to the central nervous system, damage to the lungs and kidney damage.

No allegation is intended that the present invention will prevent or alleviate all forms of the above-identified conditions, but the use will be effective against those forms of the aforementioned conditions which are attributable to or caused by the presence of lead or mercury.

For production of the isomer or isomers of IP₃ which accomplish the above objectives and which are present in the medicament according to the invention, one or more of the compounds IP₆, IP₅ or IP₄ or a natural product containing at least one of these compounds can be used as a starting material. In the cases where the starting material is a natural product, one with a content of at least 0.3%, preferably at least 1% of inositol phosphate (IP₆ + IP₅ + IP₄) is preferably chosen. Particularly suitable products are beans, bran, pollen and oil plants.

The medicament as used according to the present invention should preferably contain at least 10%, preferably at least 20%, or better yet at least 40% IP₃ calculated on the inositol content of the starting material. As high a level as possible of IP₃ in the composition is aimed at, as IP₃ has the best therapeutic effect according to experiments shown below. The IP₃ isomers present in the medicament as used according to the invention can, for example, be produced by:
1) Enzymatic breakdown starting from IP₄, IP₅ and/or IP₆.
2) Chemical hydrolysis starting from IP₄, IP₅ and/or IP₆.
3) Chemical synthesis starting, for example, with inositol, IP₁, IP₂ and phosphate.
4) Enzymatic synthesis starting for example with from inositol, IP₁, IP₂ and phosphate.
5) Microbiological production (including also hybrid DNA-techniques).
6) Chemical or enzymatic migration of inositol phosphate or
7) Chemical or enzymatic hydrolysis of substituted inositol phosphate.

A combination of two or more of the above mentioned procedures may also be used.

According to the invention a procedure where the above mentioned higher inositol phosphates IP₆, IP₅ and/or IP₄ are broken down enzymatically to IP₃ with phytase enzyme, for instance, is preferred. Phytase enzyme is normally present in all inositol phosphate containing plants and seeds. Because of this it is, according to the invention, usually not necessary to add the enzyme if a natural product is used as starting material. In the cases where the natural product has too low an enzymatic activity or when IP₆, IP₅ or IP₄ or a mixture of these is used as starting material, a phytase enzyme, for example, from bran is added.

A suitable way to treat the natural or crude starting material is to pretreat it, for instance by breakage or removal of outer membrane and removal of unwanted constituents. Thus, when using pollen the allergens should be removed. Thereafter, the material is soaked in water to make the inositol phosphate available for breaking down and to activate the enzyme. In the cases where an extra quantity or enzymes is necessary, this quantity is added at this stage. The enzyme is then allowed to act for so long a time as is necessary for the intended degree of hydrolysis to be achieved.

The hydrolysis takes place at a suitable temperature, usually 20-70°C, preferably 30-40°C and at optimal pH-level for the phytase present. In order to stop the hydrolysis at the intended level the enzyme may be destroyed or inactivated, for instance by a rapid heating of the hydrolysed starting material. This also ensures that an uncontrolled and undesired continued hydrolysis of IP₃ in the stomach will not continue when the composition is administered. In order to transfer the material to a form which is stable at storage it can suitably be freeze dried.

Yeast can be used advantageously as a source of phytase. Preferably baker's yeast is used. When using yeast essentially only one isomer of IP₃ is obtained, namely D-myo-inositol-1.2.6-triphosphate.

The above mentioned procedure, in applicable parts with possible modifications, can be used also when one or more of the compounds IP₆, IP₅ or IP₄ per se are used as starting material.

The medicament as used according to the invention comprises as a pharmaceutically active ingredient at least one isomer of inositol triphosphate (IP₃) in an amount sufficient to reduce the negative effect of lead or mercury, in the body.

It is suitable that the medicament used according to the invention exists in unit dosage form. Tablets, granulates or capsules are suitable administration forms for such unit dosage. Furthermore, tablets and granulates can easily be surface treated such as to provide an enteric coating to prevent an uncontrolled hydrolysis in the stomach and to bring about a desired absorption in the intestine. Other suitable administration forms are slow release and transdermal administration. A usual pharmaceutically acceptable additive, excipient and/or carrier can be included in the composition. The tablets or granulates can also contain a disintegrant which causes the tablets or the granulates, respectively, to disintegrate easily in the intestine. In certain cases, especially in acute situations, it is preferable to use the unit dosage in the form of a solution for intravenous administration.

The medicament can also consist as such of IP₃ solely without any additive, excipient or carrier.

If desired, the medicament can be free of other inositol phosphates IP₁, IP₂, IP₄, IP₅ and IP₆. Accordingly, the mixture of IP₃ isomers can have a purity of 90-100%, such as 93-100% or preferably 95-100%.

Alternatively, the medicament can consist of or comprise one or more specific IP₃ isomers disclosed hereinafter, each present in substantially pure form. Thus, the different isomers can be isolated from each other in substantially pure form, which means that they have a purity of 80-100%, such as 82-100% or 85-100%, preferably 90-100%. Since the isomers can be produced in pure form they can be mixed in any proportion, of course.

The production of IP₃ and the isolation of the different isomers thereof are disclosed in the U.S. patent application 788,829 filed on October 18, 1985 and the equivalent U.K. patent application 2,169,602 for instance.

It is in most cases suitable that the IP₃ isomer or isomers in the composition according to the invention be present in salt form in order not to affect the mineral balance negatively. The salt should preferably consist of a sodium, calcium, zinc or magnesium salt or a mixture of two or more of these salts. Calcium and zinc salts or mixtures of these are especially preferred. The isomer of IP₃ can also partly be present as a salt of one or more physiologically acceptable compounds in the lanthanide series; i.e. La, Ce, Pr, Nd, Pm, Sm, Eu, Gd, Tb, Dy, Ho, Er, Tm, Yb and Lu.

For the above mentioned reasons it is also an advantage if the medicament contains a surplus or an extra addition of at least one pharmaceutically acceptable salt of calcium, zinc or magnesium with a mineral acid or an organic acid. This is especially valuable for older persons who are often deficient in these minerals.

The medicament used according to the present invention can preferably also contain at least one substance containing selenium, an unsaturated fatty acid, such as gamma linoleic acid, vitamin E, vitamin C or a pharmaceutically acceptable organic acid or salt thereof, such as citrate, oxalate, malonate and tartrate. These substances also help to counteract the negative effect of lead and mercury in the body and/or to give in addition thereto, in certain cases, a desirable effect together with the IP₃ isomer in the composition. The content of selenium in the medicament is preferably such that the daily intake is about 0.7-8 µg/kg body weight preferably 0.7-3.3 µg. For vitamin E the corresponding values are about 0.1-2 mg and 0.1-1 mg, respectively.

The medicament is suitably free from penicillin.

For administration to human patients suffering from a condition caused by or attributable to the presence of lead or mercury in the body, appropriate dosages can routinely be determined by those skilled in this art by extension of the results obtained in animals at various dosages. The preferred dosage for humans falls within the range of 0.1 to 10 mg IP₃/day/kg body weight.

In animal experiments, no toxic effects were seen after administration of very high doses of IP₃, 160 mg/kg body weight by intravenous injection to mice or 1600 mg/kg body weight by intraperitoneal injection to mice.

The medicament used according to the present invention contains at least one, sometimes two or more of the following substances, which correspond to the essential IP₃-isomer or isomers mentioned above:
D-myo-inositol-1.2.6-triphosphate of the formula 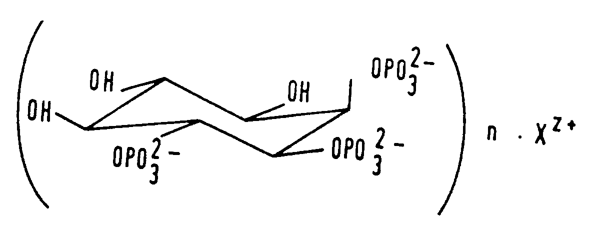
where X is hydrogen, at least one univalent, divalent or multivalent cation, or a mixture thereof, n is the number of ions, and z is the charge of the respectively ion;
D-myo-inositol-1.2.5-triphosphate of the formula 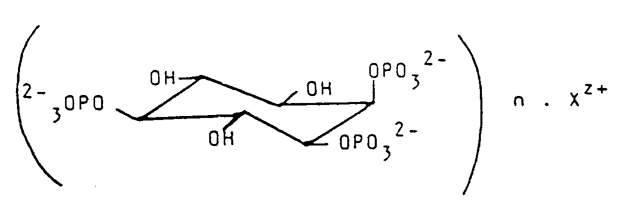
where X, n and z have the above mentioned meaning;
myo-inositol-1.2.3-triphosphate of the formula 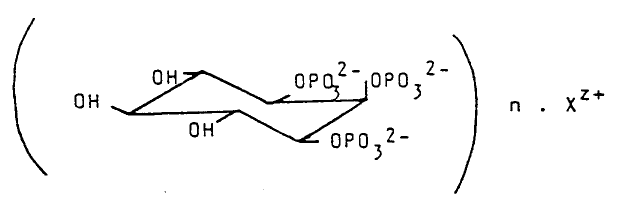
where X, n and z have the above mentioned meaning;
L-myo-inositol-1.3.4-triphosphate of the formula 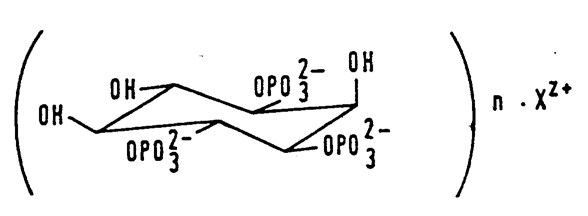
where X, n and z have the above mentioned meaning; and
D-myo-inositol-1.4.5-triphosphate of the formula 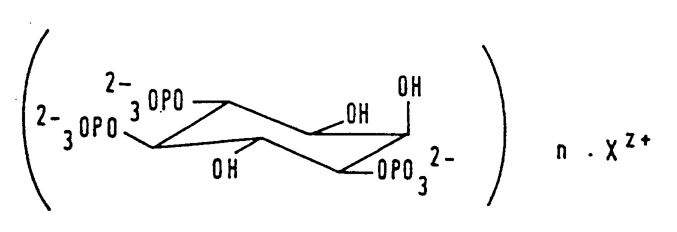
where X, n and z have the above mentioned meaning.

In each of the above formulas n ranges between 6 to 1 inclusive and z ranges from 1 to 6 inclusive. Preferably, n is between 3 to 6 inclusive and z is 3, 2 or 1. Of above isomers D-myo-inositol-1.2.6-triphosphate is preferred.

IP₃ may be the sole pharmaceutically active ingredient in the medicament. However, also other pharmaceutically active ingredients can be present therein. The amount of IP₃ should then constitute 5 to 95 or 15 to 80, such as 25 to 60 per cent by weight of said active ingredients.

Moreover, the medicament can be a multi-vitamin unit containing 2 to 60, such as 2 to 40 or preferably 2 to 25 per cent by weight of IP₃ based on the total weight of pharmaceutically active ingredients.

The medicament usually contains 0.01-1.5 g, such as 0.05-1.3 or preferably 0.1-1 g of IP₃.

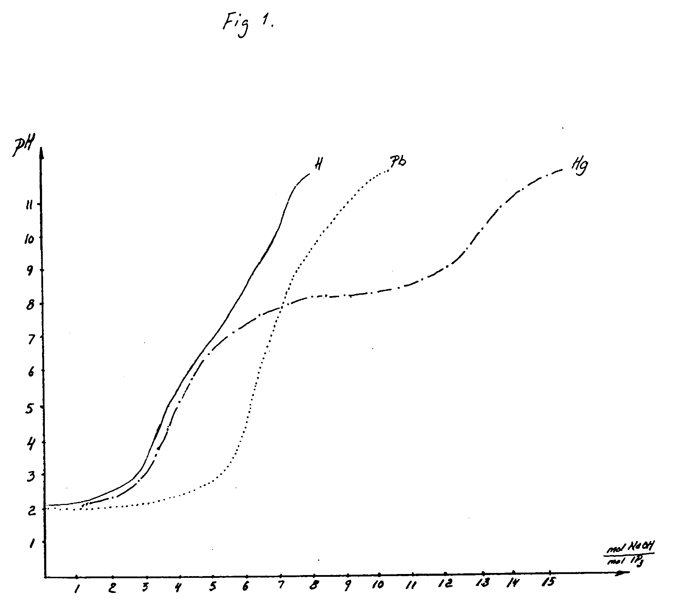

The invention is further explained below in connection with enclosed Figure 1 and embodiment examples of which example 1 shows experiments relating to the relative complex constants for lead or mercury respectively to IP₃. Example 2 illustrates an experiment concerning the complex constant of lead to IP₃. Example 3 relates to experiments on structural changes of the cell membranes in the presence of Pb, and the use of IP₃ to counteract such changes.

Example 4 illustrates experiments on the damaging effect of Pb and Hg on erythrocyte cell membrane. Examples 5-11 show production of IP₃ and separation thereof into different isomers. In example 12 the manufacture of a solution of a potassium salt of D-myo-inositol-1.2.6-triphosphate for injection is shown. Example 13 finally describes manufacture of tablets of a calcium salt of D-myo-inositol-1.2.6-triphosphate. Fig. 1 shows the result of the experiments according to example 1.

### Example 1

The relative complex constants for D-myo-inositol-1.2.6-triphosphate (IP₃) and lead and mercury respectively were determined.

A solution consisting of 4 mM IP₃ was titrated with 100 mM NaOH. Similar titrations were performed in the presence of Pb and, Hg (12 mM).

A strong metal complex will result in a lowering of pH at a certain amount NaOH added. Figure 1 shows the performed titrations and the relative metal binding properties. At pH 9, the binding properties are as follows: Pb < Hg.

As can be seen IP₃ binds each of the metals very strongly.

### Example 2

The binding constant for the complex D-myo-inositol-1.2.6-triphosphate - lead was determined with NMR.

By comparing the signal intensities and ratios in ³²P-NMR as a function of the amount of lead added to an inositoltriphosphate solution the binding constant (K) for the complex was calculated to be 10⁸.

### Example 3

The structure of the cell membrane is very essential for the proper function of the cell. It is known that the presence of Fe (II) in liposomes (phospholipids from oxbrain) damages the cell membrane. One measurement of the damage is the determination of the lipidperoxides formed when the metal is added to the preparation.

In this example, the synergistic damaging effects of the cell membrane when Pb is added to the preparations together with Fe (II), were investigated. Furthermore the preventive effect of the presence of D-myo-inositol-1.2.6-triphosphate (IP₃) was evaluated.

### Reaction mixture

| | |
|---|---|
| Clark-Lubs buffer pH 5.5 | 40 mM |
| Liposomes, Sigma type VII | 1 mg/ml |
| IP₃ | 1.0 mM |
| (NH₄)₂ Fe(SO₄)₂ | 0.1 mM |
| Pb²⁺ | 0.4 mM |

The reaction mixture (1.0 ml) was incubated for 2 hours at 37°C. After incubation 0.5 ml of thiobarbituric acid and 0.5 ml 25% HCl were added and the mixture was heated at 100°C for 20 minutes. The amount of lipid peroxides was measured by measuring the absorbance at 532 nm.

| Metal concentration (mM) | | | | |
|---|---|---|---|---|
| Experiment | Fe | Pb | IP₃ | Absorbance |
| 1 | - | - | - | 0.044 |
| 2 | 0.1 | - | - | 0.47 |
| 3 | 0.1 | - | 1.0 | 0.25 |
| 4 | 0.1 | 0.4 | - | 0.54 |
| 5 | 0.1 | 0.4 | 1.0 | 0.37 |

The structural change of the cell membranes caused by Fe (II) (Experiment 2) was strongly increased by the presence of Pb (Experiment 4). This effect was counteracted by IP₃ (Experiment 5).

A disfunction of the cell caused by disturbances in the structure of the cell membrane can be linked to many diseases.

### Example 4

When erythrocytes are treated with hydrogen peroxide they undergo lipidperoxidation which damages the structure of the cell membrane and the function of the erythrocytes.

In this example the .increased damaging effects of the erythrocyte cell membrane when Pb or Hg was added to the preparations, were investigated. Furthermore, the preventive effect of the presence of D-myo-inositol-1.2.6-triphosphate (IP₃) was evaluated.

As described in Example 3 the absorbance at 532 nm was used as a measurement of the amount of lipidperoxides formed and consequently the damage of the erythrocyte cell membrane.

| Addition to the reaction mixture (containing erythrocytes, buffer pH 7.4 and H₂O₂). | | |
|---|---|---|
| Experiment | | Absorbance |
| 1 | - | 0.16 |
| 2 | 3mM IP₃ | 0.10 |
| 3 | 0.4 mM Pb²⁺ | 0.22 |
| 4 | 0.4 mM Pb + 3mM IP₃ | 0.13 |
| 5 | 0.4 mM Hg²⁺ | 0.26 |
| 6 | 0.4 mM Hg²⁺ + 3mM IP₃ | 0.15 |

The damage to the cell membranes of the erythrocytes was increased when Pb (Experiment 3) or Hg (Experiment 5) was added.

These effects were strongly counteracted by the presence of IP₃ (Experiments 4 and 6

### Example 5

Hydrolysis of sodium phytate with wheat phytase and fractionation of a mixture of inositolphosphates.

A 1.6 gram quantity of sodium phytate (from corn, Sigma® Chemical Co.) was dissolved in 650 ml sodium acetate buffer, pH 5.2. 2.79 grams of wheat phytase (EC 3.1.3.26, 0.015 IU/mg, from Sigma® Chemical Co.) was added and the mixture was incubated at 38°C.

The dephosphorylation was followed by determining the inorganic phosphorus released. After 3 hours when 50% inorganic phosphorus was liberated the hydrolysis was stopped by adding 30 ml ammonia to pH 12. A liquid mixture containing inositolphosphates was obtained.

350 ml of the mixture was passed through an ion-exchange column (Dowex® 1, chloride form, 25 mm x 250 mm) and eluted with a linear gradient of hydrochloric acid (0-0.7 N HCl). Aliquots of eluted fractions were completely hydrolyzed in order to determine the contents of phosphorus and inositol. The peaks correspond to different inositolphosphates, i.e. a peak with the ratio of phosphorus to inositol of three to one consists of inositoltriphosphate etc. Two fractions with the ratio of phosphorus to inositol of three to one were obtained.

### Example 6

### Fractionation of inositoltriphosphates.

100 ml of the first fraction obtained in Example 5 with a phosphorus/inositol ratio of three to one was neutralized and precipitated as a bariumsalt after addition of a 10% excess of 0.1 M bariumacetate solution. 600 mg of the precipitated salt was dissolved in 50 ml diluted hydrochloric acid. The solution was separated on an ion-exchange column (Dowex® 1, chloride form, 25 mm x 2500 mm) with diluted hydrochloric acid as eluent. Aliquots of eluted fractions were analyzed for phosphorus. Three peaks consisting of isomers of inositoltriphosphates could be seen.

### Example 7

### Structural determination of isomers of inositoltriphosphates with NMR.

The three peaks obtained in Example 6 were analyzed by H-NMR. Data show that the peaks consist of myo-inositol-1.2.6-triphosphate, myo-inositol-1.2.3-triphosphate and myo-inositol-1.3.4-triphosphate respectively.

The second fraction obtained in Example 5 with a phosphorus/inositol ratio of three to one was analyzed by H-NMR. Data show that the fraction consists of myo-inositol-1.2.5-triphosphate.

### Example 8

### Determination of optical isomers of inositoltriphosphate.

20 mg of the compounds determined with NMR according to Example 7 to be myo-inositol-1.2.6-triphosphate and myo-inositol-1.3.4-triphosphate were further chromatographed on a chiral column based on acetylated cellulose (20 mm x 300 mm from Merck) with a mixture of ethanol and water as eluent. The fractions were analyzed with a polarimeter. Each compound consists of one optical isomer, D-myo-inositol-1.2.6-triphosphate and L-myo-inositol-1.3.4-triphosphate respectively.

### Example 9

### Hydrolysis of sodium phytate with baker's yeast and fractionation of a mixture of inositolphosphates.

A 0.7 gram quantity of sodium phytate (from corn, Sigma® Chemical Co.) was dissolved in 600 ml sodium acetate buffer pH 4.6. 50 grams of baker's yeast from Jästbolaget, Sweden (dry substance: 28%, nitrogen content: 2%, phosphorus content: 0.4%) were added with stirring and incubation was continued at 45°C. The dephosphorylation was followed by determining the inorganic phosphorus released. After 7 hours when 50% inorganic phosphorus was liberated the hydrolysis was stopped by adding 30 ml of ammonia to pH 12. The suspension was centrifuged and the supernatent was collected.

400 ml of the supernatent was passed through an ion-exchange column (Dowex® 1, chloride form, 25 mm x 250 mm) and eluted with a linear gradient of hydrochloric acid (0-0.7 N HCl).

Aliquots of eluted fractions were completely hydrolyzed in order to determine the contents of phosphorus and inositol. The peaks correspond to different inositolphosphates, i.e. a peak with the ratio of phosphorus to inositol of three to one consists of inositoltriphosphates etc.

### Example 10

### Structural determination of isomers of inositoltriphosphate.

The fraction obtained in Example 9 with a phosphorus/inositol ratio of three to one was neutralized and evaporated before analysis with H-NMR. Data show that the peak consists of myo-inositol-1.2.6-triphosphate.

### Example 11

### Determination of optical isomers of myo-inositol-triphosphate.

The same method was used as described in Example 8 with the difference that 10 mg of the compound determined with NMR according to Example 10 was analyzed. The compound consists of one optical isomer, D-myo-inositol-1.2.6-triphosphate.

### Example 12

### Solution of potassium salt of D-myo-inositol-1.2.6-triphosphate for injection.

0.5 g of the potassium salt of IP₃ and 0.77 g NaCl were dissolved in 98.73 ml of water for injection to form a solution suitable for injection into a person or an animal.

### Example 13

### Tablets of calcium salt of D-myo-inositol-1.2.6-triphosphate.

Tablets of the calcium salt of D-myo-inositol-1.2.6-triphosphate were produced in the following way. 50 g calcium salt of D-myo-inositol-1.2.6-triphosphate, 132 g lactose and 6 g acacia gum were mixed. Purified water was then added to the mixture, whereupon the mixing was continued until a suitable consistency was obtained. The mixture was sieved and dried. Then the mixture was blended with 10 g talcum and 2 g magnesium stearate. The mixture was compressed into tablets each weighing 200 mg.

For purposes of further understanding the invention, formulas are given below of the IP₃ isomers of the invention. Formulas are also given for IP₆, IP₅, IP₄ and IP₂.

The lower phosphate esters of myo-inositol are named depending on where the phosphoric acid groups are situated on the inositol ring, with the numbering giving as low position numbers as possible. L and D stand for clock-wise and counterclock-wise counting respectively, and are used depending on which result gives the lowest position number. The carbon atom which has an axial phosphoric acid group always has the position number 2. The structural formulas below are simplified to the acid form.

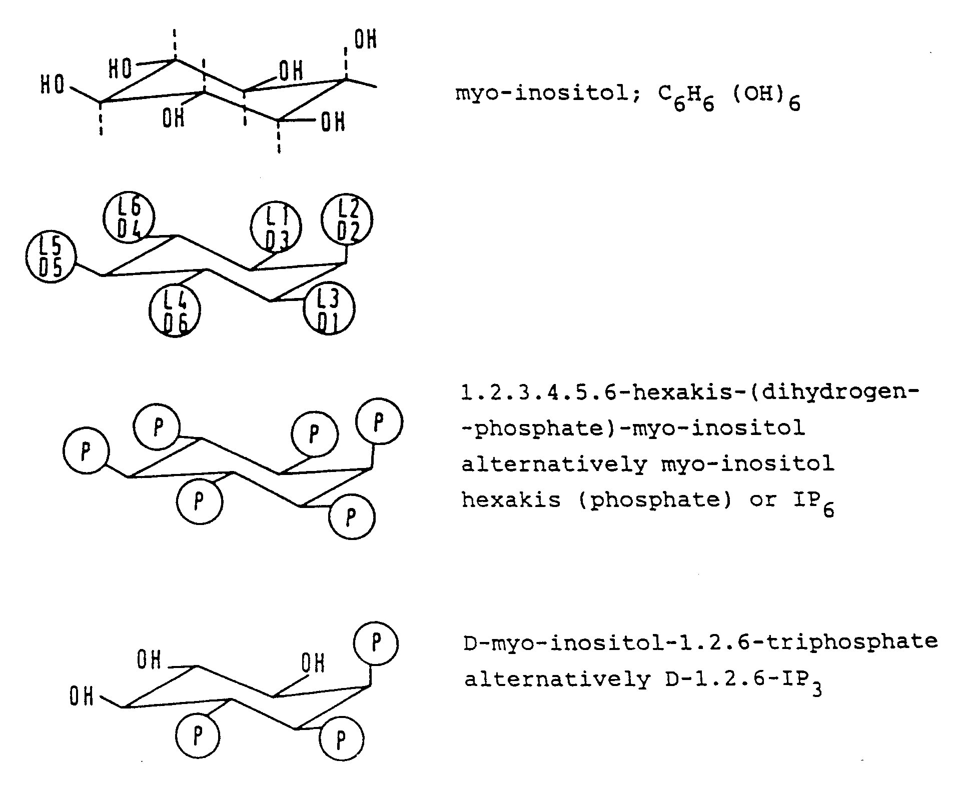

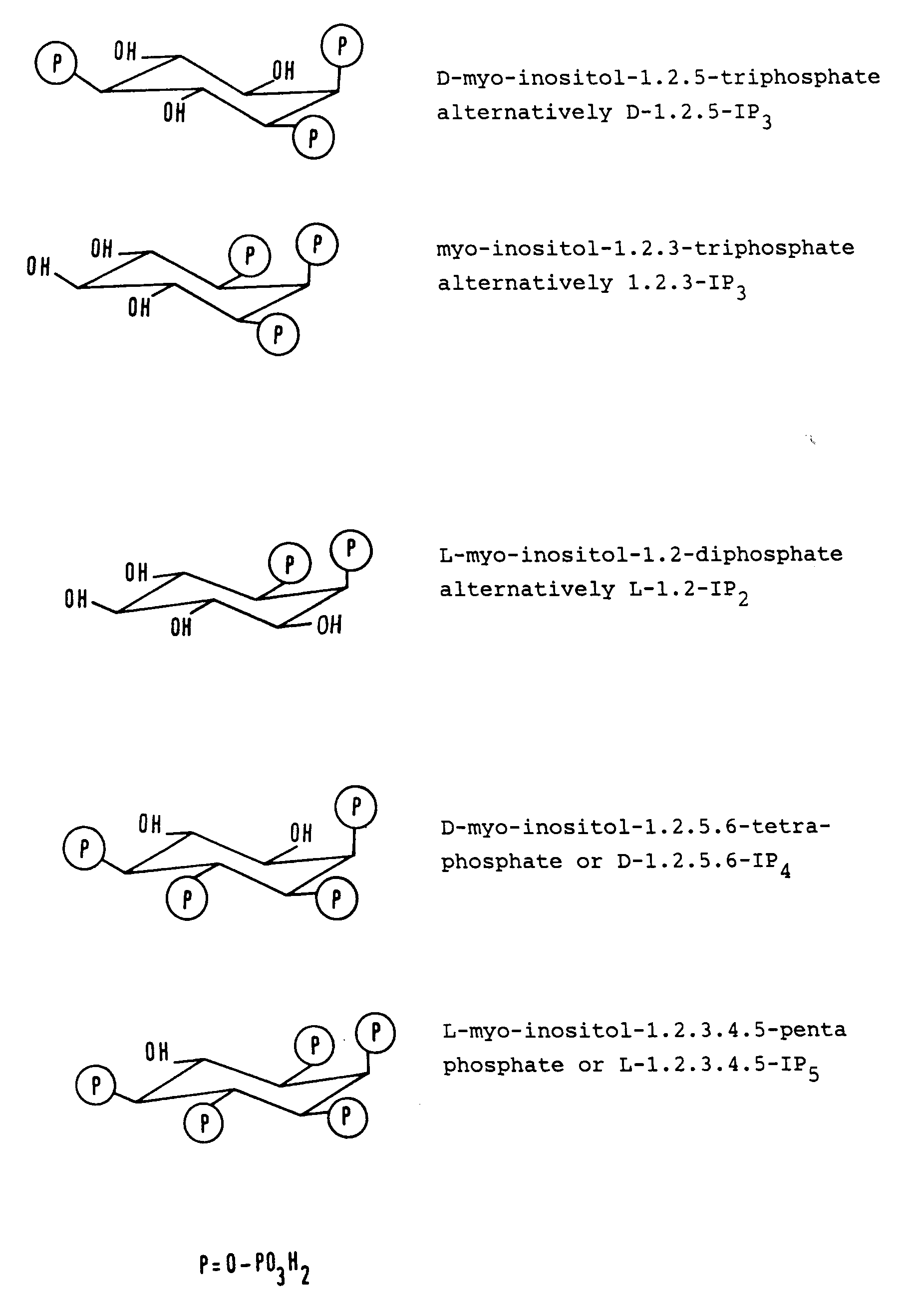

## Claims

1. The use of inositoltriphosphate (IP₃) for the preparation of a medicament effective against conditions which are attributable to or caused by the presence of lead or mercury.

2. The use of inositol triphosphate for the prepartion of medicaments for preventing or alleviating metal intoxication caused by lead or mercury.

3. The use according to claim 1 or 2, wherein said inositoltriphosphate is in salt form.

4. The use according to claim 3, wherein said inositoltriphosphate salt is a salt of sodium, calcium, zinc or magnesium or a mixture of two or more thereof.

5. The use according to any one of claims 1-4, wherein the medicament is in tablet or granulated form.

6. The use according to any one of claims 1-4, wherein the medicament is in the form of a solution.

7. The use according to any one of claims 1-6, wherein the medicament is further comprising a pharmaceutically acceptable salt of a mineral acid or an organic acid with at least one of calcium, zinc or magnesium.

8. The use according to any one of claims 1-7, wherein the medicament is further comprising at least one additive selected from a selenium compound, an unsaturated fatty acid such as gamma linolenic acid, vitamin E, vitamin C and a pharmaceutically acceptable organic acid or salt thereof.

9. The use according to claim 8, wherein said salt is a citrate, oxalate, malonate or tartrate.

10. The use according to any one of claims 1-9, wherein the medicament is free from penicillin.

11. The use according to any one of claims 1-10, wherein the medicament is containing at least one other pharmaceutically active ingredient in addition to IP₃.

12. The use according to claim 11, wherein the amount of IP₃ is in the range of 5-95 per cent by weight of the active ingredients.

13. The use according to claim 11, wherein the medicament is a multi-vitamin unit containing 2-60 per cent by weight of IP₃ based on the total weight of pharmaceutically active ingredients.

14. The use according to any one of claims 1-13, wherein the medicament is containing 0.01-1.5 g of IP₃.

15. The use according to any one of claims 1-14, wherein IP₃ has the formula 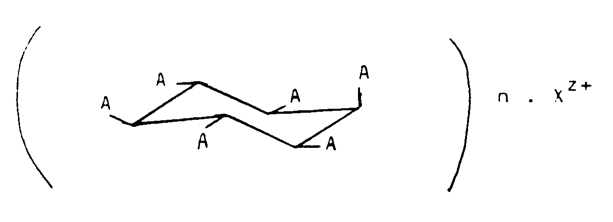 where three A is OH and three A is OPO₃²⁻,
X is hydrogen and/or at least one univalent, divalent or multivalent cation; n is the number of ions; and z is the charge of the respective ions.

16. The use according to claim 15, wherein n ranges between 6 to 1 inclusive, z ranges between 1 to 6 inclusive and n is preferably between 3 to 6 inclusive and z is 3, 2 or 1.

17. The use according to claim 15, wherein said IP₃ comprises D-myo-inositol-1.2.6-triphosphate with the formula 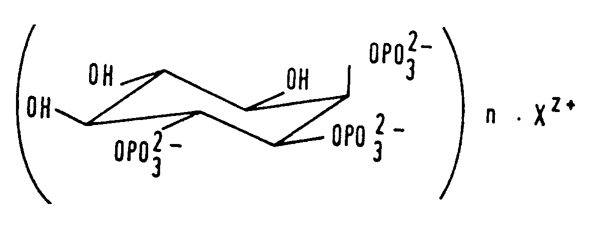 where X is hydrogen or at least one univalent, divalent or multivalent cation; n is the number of ions; and z is the charge of the respective ion.

18. The use according to claim 15, wherein said IP₃ comprises D-myo-inositol-1.2.5-triphosphate with the formula 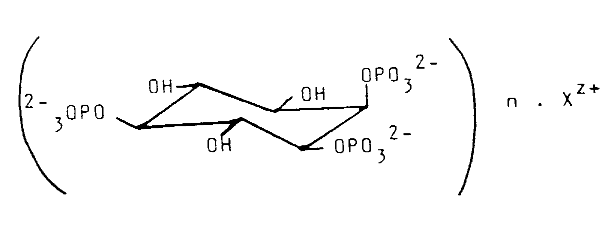 where X is hydrogen or at least one univalent, divalent or multivalent cation; n is the number of ions; and z is the charge of the respective ion.

19. The use according to claim 15, wherein said IP₃ comprises myo-inositol-1.2.3-triphosphate with the formula 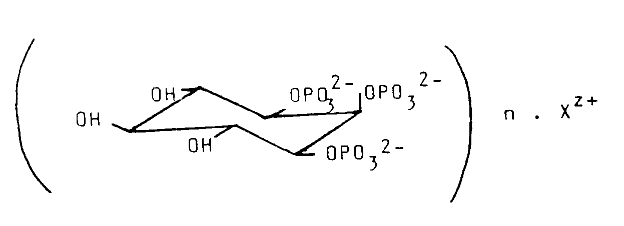 where X is hydrogen or at least one univalent, divalent or multivalent cation; n is the number of ions; and z is the charge of the respective ion.

20. The use according to claim 15, wherein said IP₃ comprises L-myo-inositol-1.3.4-triphosphate with the formula 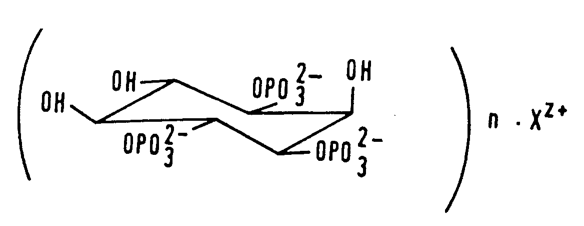 where X is hydrogen and/or at least one univalent, divalent or multivalent cation; n is the number or ions; and z is the charge of the respective ion.

21. The use according to claim 15, wherein said IP₃ comprises D-myo-inositol-1.4.5-triphosphate with the formula 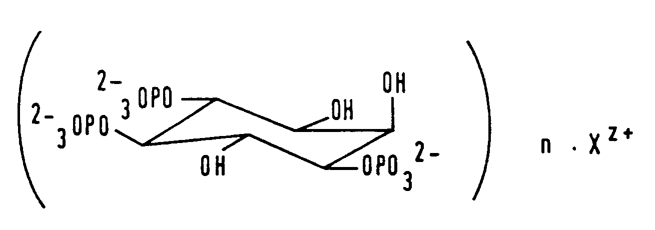 where X is hydrogen and/or at least one univalent, divalent or multivalent cation; n is the number of ions; and z is the charge of the respective ion.

22. The use according to any one of claims 1-21, wherein the medicament is in a pharmaceutical unit dosage form and further comprising a pharmaceutically acceptable carrier, excipient or additive.

## Patentansprüche

1. Verwendung von Inosittriphosphat (IP₃) zur Herstellung eines Arzneimittels, das gegen Erkrankungen, welche der Gegenwart von Blei oder Quecksilber zuzuschreiben sind oder durch diese verursacht werden, wirksam ist.

2. Verwendung von Inosittriphosphat zur Herstellung eines Arzneimittels zur Verhinderung oder Milderung einer durch Blei oder Quecksilber verursachten Metallvergiftung.

3. Verwendung nach Anspruch 1 oder 2, wobei das Inosittriphosphat in Salzform vorliegt.

4. Verwendung nach Anspruch 3, wobei das Inosittriphosphatsalz ein Salz von Natrium, Kalzium, Zink oder Magnesium oder von einer Mischung aus zwei oder mehreren der genannten ist.

5. Verwendung nach einem der Ansprüche 1 bis 4, wobei das Arzneimittel als Tablette oder in granulierter Form vorliegt.

6. Verwendung nach einem der Ansprüche 1 bis 4, wobei das Arzneimittel in Form einer Lösung vorliegt.

7. Verwendung nach einem der Ansprüche 1 bis 6, wobei das Arzneimittel außerdem ein pharmazeutisch akzeptables Salz einer Mineralsäure oder einer organischen Säure mit mindestens einem der Elemente Kalzium, Zink oder Magnesium enthält.

8. Verwendung nach einem der Ansprüche 1 bis 7, wobei das Arzneimittel ferner mindestens einen Zusatzstoff, ausgewählt aus einer Selenverbindung, einer ungesättigten Fettsäure wie z. B. gamma-Linolensäure, Vitamin E, Vitamin C und einer pharmazeutisch akzeptablen organischen Säure oder einem Salz derselben, enthält.

9. Verwendung nach Anspruch 8, wobei das Salz ein Citrat, Oxalat, Malonat oder Tartrat ist.

10. Verwendung nach einem der Ansprüche 1 bis 9, wobei das Arzneimittel penicillinfrei ist.

11. Verwendung nach einem der Ansprüche 1 bis 10, wobei das Arzneimittel zusätzlich zu IP₃ mindestens ein anderes pharmazeutisch aktives Ingredienz enthält.

12. Verwendung nach Anspruch 11, wobei die Menge an IP₃ im Bereich von 5 bis 95 Gew.-%, bezogen auf die aktiven Ingredienzien, liegt.

13. Verwendung nach Anspruch 11, wobei das Arzneimittel eine Multivitamin-Einheit ist, die 2 bis 60 Gew.-% IP₃, bezogen auf das Gesamtgewicht der pharmazeutisch aktiven Ingredienzien, enthält.

14. Verwendung nach einem der Ansprüche 1 bis 13, wobei das Arzneimittel 0,01 bis 1,5 g IP₃ enthält.

15. Verwendung nach einem der Ansprüche 1 bis 14, wobei IP₃ die Formel 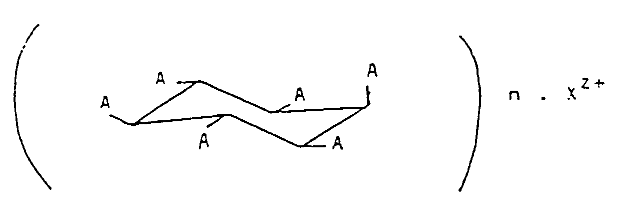 hat, worin drei A gleich OH sind und drei A OPO₃²⁻ sind; X Wasserstoff und/oder mindestens ein einwertiges, zweiwertiges oder mehrwertiges Kation ist; n die Anzahl der Ionen ist; und Z die Ladung der jeweiligen Ionen ist.

16. Verwendung nach Anspruch 15, wobei n im Bereich von 6 bis einschließlich 1 liegt; z im Bereich von 1 bis 6 (einschließlich) liegt, und n vorzugsweise 3 bis 6 (einschließlich) ist; und z gleich 3, 2 oder 1 ist.

17. Verwendung nach Anspruch 15, wobei das IP₃ D-myo-Inosit-1,2,6-triphosphat der Formel 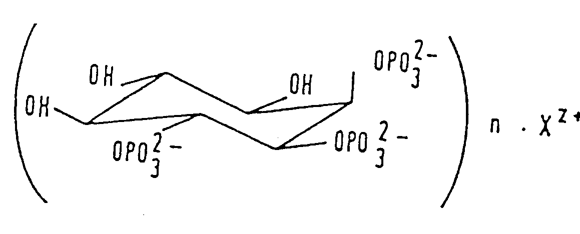 umfaßt, worin X Wasserstoff oder mindestens ein einwertiges, zweiwertiges oder mehrwertiges Kation ist; n die Anzahl der Ionen ist; und z die Ladung des jeweiligen Ions ist.

18. Verwendung nach Anspruch 15, wobei das IP₃ D-myo-Inosit-1,2,5-triphosphat der Formel 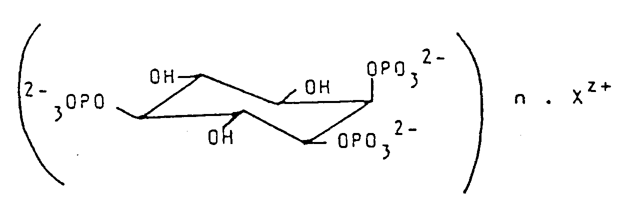 umfaßt, worin X Wasserstoff oder mindestens ein einwertiges, zweiwertiges oder mehrwertiges Kation ist; n die Anzahl der Ionen ist; und z die Ladung des jeweiligen Ions ist.

19. Verwendung nach Anspruch 15, wobei das IP₃ myo-Inosit-1,2,3- triphosphat der Formel 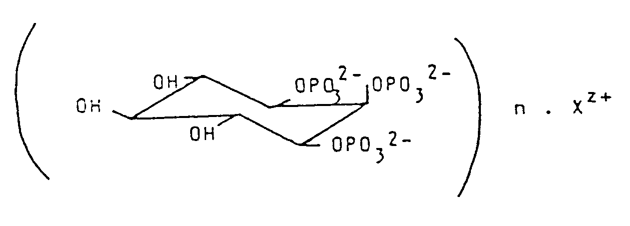 umfaßt, worin X Wasserstoff oder mindestens ein einwertiges, zweiwertiges oder mehrwertiges Kation ist; n die Anzahl der Ionen ist; und z die Ladung des jeweiligen Ions ist.

20. Verwendung nach Anspruch 15, wobei das IP₃ L-myo-Inosit-1,3,4-triphosphat der Formel 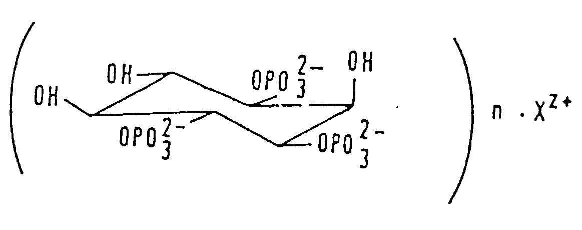 umfaßt, worin X Wasserstoff und/oder mindestens ein einwertiges, zweiwertiges oder mehrwertiges Kation ist; n die Anzahl der Ionen ist; und z die Ladung des jeweiligen Ions ist.

21. Verwendung nach Anspruch 15, wobei das IP₃ D-myo-Inosit-1,4,5-triphosphat der Formel 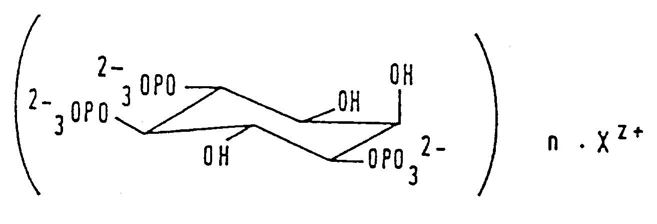 umfaßt, worin X Wasserstoff und/oder mindestens ein einwertiges, zweiwertiges oder mehrwertiges Kation ist; n die Anzahl der Ionen ist; und z die Ladung des jeweiligen Ions ist.

22. Verwendung nach einem der Ansprüche 1 bis 21, wobei das Arzneimittel in Form einer pharmazeutischen Dosierungseinheit vorliegt und außerdem einen pharmazeutisch akzeptablen Träger, eine pharmazeutisch akzeptable Arzneimittelträgersubstanz oder einen pharmazeutisch akzeptablen Zusatzstoff enthält.

## Revendications

1. Utilisation de l'inositoltriphosphate (IP₃) pour la préparation d'un médicament efficace contre les états qu'on peut attribuer à la présence de plomb ou de mercure ou qui peuvent être provoqués par cette présence.

2. Utilisation d'inositoltriphosphate pour la préparation de médicaments pour éviter ou soulager l'intoxication par des métaux provoquée par le plomb ou le mercure.

3. Utilisation selon la revendication 1 ou 2, dans laquelle ledit inositoltriphosphate est sous la forme d'un sel.

4. Utilisation selon la revendication 3, dans laquelle ledit sel d'inositoltriphosphate est un sel de sodium, de calcium, de zinc ou de magnésium, ou un mélange de deux ou plusieurs de ceux-ci.

5. Utilisation selon l'une quelconque des revendications 1-4, dans laquelle le médicament se présente sous la forme de comprimés ou de granules.

6. Utilisation selon l'une quelconque des revendications 1-4, dans laquelle le médicament se présente sous la forme d'une solution.

7. Utilisation selon l'une quelconque des revendications 1-6, dans laquelle le médicament comprend en outre un sel pharmaceutiquement acceptable d'un acide minéral ou d'un acide organique, avec au moins un sel de calcium, ou de zinc ou de magnésium.

8. Utilisation selon l'une quelconque des revendications 1-7, dans laquelle le médicament comprend en outre au moins un additif choisi parmi un composé du sélénium, un acide gras insaturé tel que l'acide gamma linolénique, la vitamine E, la vitamine C et un acide organique pharmaceutiquement acceptables ou sel de celui-ci.

9. Utilisation selon la revendication 8, dans laquelle ledit sel est un citrate, un oxalate, un malonate ou un tartrate.

10. Utilisation selon l'une quelconque des revendications 1-9, dans laquelle le médicament est exempt de pénicilline.

11. Utilisation selon l'une quelconque des revendications 1-10, dans laquelle le médicament contient au moins un autre ingrédient pharmaceutiquement actif en plus de l'IP₃.

12. Utilisation selon la revendication 11, dans laquelle la quantité de l'IP₃ est dans la gamme 5-95 % en poids des ingrédients actifs.

13. Utilisation selon la revendication 11, dans laquelle le médicament est une unité multi-vitamine contenant 2-60 % en poids d'IP₃ par rapport au poids total des ingrédients pharmaceutiquement actifs.

14. Utilisation selon l'une quelconque des revendications 1-13, dans laquelle le médicament contient 0,01-1,5 g d'IP₃.

15. Utilisation selon l'une quelconque des revendications 1-14, dans laquelle l'IP₃ a la formule : 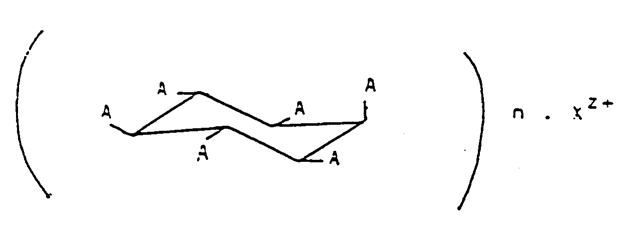 dans laquelle trois A sont OH et trois A sont OPO₃²⁻, X est un atome d'hydrogène et/ou au moins un cation univalent, divalent ou multivalent; n est le nombre d'ions; et z est la charge des ions respectifs.

16. Utilisation selon la revendication 15, dans laquelle n est compris entre 6 et 1 inclus, z est compris entre 1 et 6 inclus et n est de préférence compris entre 3 et 6 inclus et z est 3, 2 ou 1.

17. Utilisation selon la revendication 15, dans laquelle ledit IP₃ comprend du D-myo-inositol-1,2,6-triphosphate avec la formule : 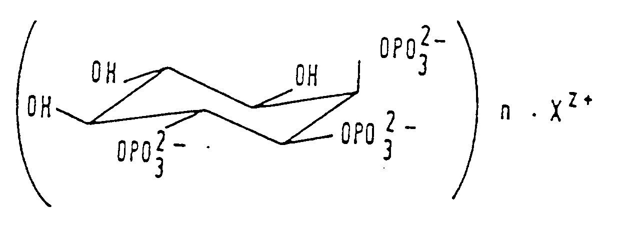 dans laquelle X est un atome d'hydrogène ou au moins un cation univalent, divalent ou multivalent; n est le nombre d'ions; et z est la charge de l'ion respectif.

18. Utilisation selon la revendication 15, dans laquelle ledit IP₃ comprend du D-myo-inositol-1,2,5-triphosphate avec la formule : 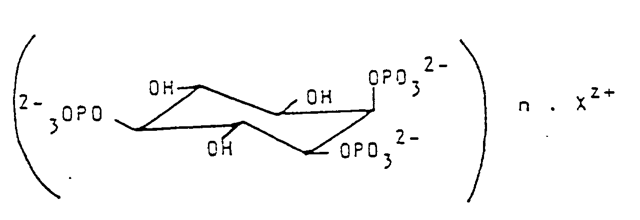 dans laquelle X est un atome d'hydrogène ou au moins un cation univalent, divalent ou multivalent; n est le nombre d'ions; et z est la charge de l'ion respectif.

19. Utilisation selon la revendication 15, dans laquelle ledit IP₃ comprend du D-myo-inositol-1,2,3-triphosphate avec la formule : 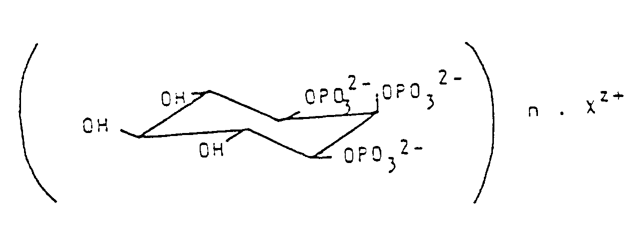 dans laquelle X est un atome d'hydrogène ou au moins un cation univalent, divalent ou multivalent; n est le nombre d'ions; et z est la charge de l'ion respectif.

20. Utilisation selon la revendication 15, dans laquelle ledit IP₃ comprend du L-myo-inositol-1,3,4-triphosphate avec la formule : 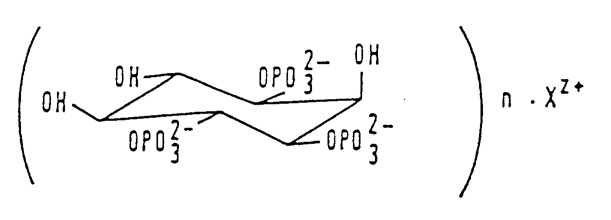 dans laquelle X est un atome d'hydrogène et/ou au moins un cation univalent, divalent ou multivalent; n est le nombre d'ions; et z est la charge de l'ion respectif.

21. Utilisation selon la revendication 15, dans laquelle ledit IP₃ comprend du D-myo-inositol-1,4,5-triphosphate avec la formule : 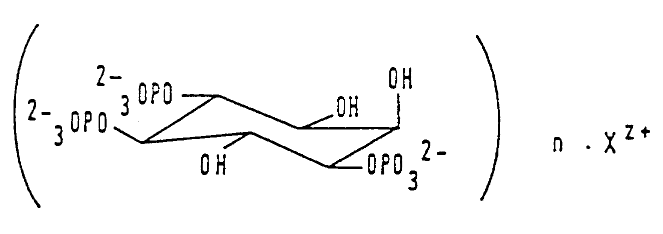 dans laquelle X est un atome d'hydrogène et/ou au moins un cation univalent, divalent ou multivalent; n est le nombre d'ions; et z est la charge de l'ion respectif.

22. Utilisation selon l'une quelconque des revendications 1-21, dans laquelle le médicament se présente sous la forme d'une dose pharmaceutique unitaire et comprend en outre un support, un excipient ou un additif pharmaceutiquement acceptables.
